# EUROPÄISCHE PATENTSCHRIFT

(11) **EP 2 496 279 B1**
(45) Veröffentlichungstag und Bekanntmachung des Hinweises auf die Patenterteilung: **22.01.2014**
(21) Anmeldenummer: 10771784.5
(22) Anmeldetag: 04.11.2010
(51) Int. Cl.: A61M 1/10, F04B 43/12

(54) **SCHLAUCHPUMPE**
PERISTALTIC PUMP
POMPE PERISTALTIQUE

(30) Priorität: 04.11.2009 DE 102009046406
(43) Veröffentlichungstag der Anmeldung: 12.09.2012
(73) Patentinhaber: Fresenius Medical Care Deutschland GmbH, 61352 Bad Homburg (DE)
(72) Erfinder: BRANDL, Matthias, 97631 Bad Königshofen (DE); PRINZ, Martin, 97762 Hammelburg (DE); PLEINER, Franz, 97230 Estenfel (DE)
(74) Vertreter: Nordmeyer, Philipp Werner
(86) Internationale Anmeldenummer: PCT/EP2010/066777
(87) Internationale Veröffentlichungsnummer: WO 2011/054890

(56) Entgegenhaltungen:
- US-A1- 2005 010 077
- US-A1- 2008 213 113

## Beschreibung

Die Erfindung bezieht sich auf eine Schlauchpumpe gemäß dem Oberbegriff des Anspruchs 1.

Im medizinischen Bereich werden Schlauchpumpen verwendet, um extrakorporal Flüssigkeiten zu fördern oder mengengenau bereitzustellen. Eine Anwendung für Schlauchpumpen findet sich bei Dialysegeräten, bei denen Dialysat des Dialysatkreislaufs, Dialysierflüssigkeiten und/oder Blut gepumpt werden muss. Bei einer Schlauchpumpe wird ein flexibler Schlauch entlang eines zylindrischen Innendurchmessers gelegt und durch radial nach außen wirkende Andruckrollen lokal verschlossen oder okkludiert und durch die Bewegung der angetriebenen Rollen entlang des Schlauches wird verschlossene Stelle verlagert und so die Fluidförderung realisiert. Ein Vorteil von Schlauchpumpen liegt in der relativ guten Dosierbarkeit. Da nur der Schlauch mit dem geförderten Fluid in Kontakt kommt, lässt sich eine Schlauchpumpe schnell und kostengünstig durch den Austausch des Schlauchs reinigen.

Aufgrund des Kontakts von den Rollen zu dem Schlauch, verbunden mit einem Walken und von Reibung, kann es am Schlauch zu triboelektrischen Aufladungen kommen. Zu derartigen Aufladungen neigen vor allem Kunststoffoberflächen. Dabei werden durch die Berührung und Reibung der Oberflächen des Stators, des Rotors, also seinen angetriebenen Rollen mit dem Schlauch Ladungen aufgebaut und ausgetauscht und beim Entfernen der Rollen vom Schlauch können diese nicht schnell genug ausgeglichen werden und verbleiben als elektrostatische Ladungen auf den jeweiligen Oberflächen. Die Ladungen entstehen dadurch, dass zuerst der Schlauch durch die Andruckkraft der Rollen sowohl an das Rollbett, als der entsprechenden Kontaktfläche des Stators, und die Rollen angedrückt wird. Beim Weiterrollen hebt die jeweilige Rolle von dem Schlauchabschnitt ab, so dass es zu der beschriebenen elektrostatischen Aufladung sowohl des Schlauchs als auch des Rollbetts und der Rollen kommen kann. Ferner heben die Rollen im Bereich der Ausführung des Schlauchs aus der Schlauchpumpe komplett von dem Schlauch ab. Wenn der Rotor weiterdreht und die jeweilige Rolle im Bereich der Einführung des Schlauchs wieder mit dem Schlauch in Kontakt kommt, so können elektrostatische Ladungen, welche sich auf der Rolle angesammelt hatten, auf den Schlauch übertragen und so einen entsprechenden Störimpuls verursachen. Diese Ladungen führen zu einer elektrostatischen Aufladung des Schlauchs und insbesondere dessen Außenoberfläche. Die Ladungstrennung wird entsprechend dem triboelektrischen Effekt nämlich dadurch bewirkt, dass unterschiedliche Materialien unterschiedliche Elektronenaffinität aufweisen und bei der Trennung von entsprechenden Materialien sich die Elektronen nicht ausreichend frei bewegen können um so einen Ladungsausgleich zu bewirken.

Neben der Erzeugung der Ladungen im Kontakt von der Rolle zu dem Schlauch, kann es auch zwischen dem Schlauch und dem Rollbett zu entsprechenden elektrostatischen Ladungsbildungen kommen. Wenn nämlich beim Weiterwandern des Rotors die jeweilige Rolle vom entsprechenden Schlauchabschnitt abhebt, so bewirken die elastischen Rückstellkräfte innerhalb des Schlauchs seine Rückformung in seine runde Grundform, so dass der Schlauch nicht mehr im flächigem, sondern nur noch in Linienkontakt zu seinem Rollbett steht. Dies ist ein teilweises Abheben des Schlauchs von dem Schlauchbett, wodurch entsprechend elektrostatische Aufladungen entstehen können.

Im medizinischen Bereich werden Diagnosegeräte mit hochohmigen Messeingängen, wie z.B. EKG-Geräte, verwendet und deren Messergebnisse können durch die elektrostatischen Aufladungen des Schlauchs gestört oder verfälscht werden. Dieses an sich bekannte Problem wird durch die Empfehlung der zuständigen Bundesbehörde BFARM einen gemeinsamen Potentialausgleich der Geräte, also der Pumpe und dem Diagnosegerät zu schaffen, gemindert ohne jedoch beseitigt zu werden. Diese elektrostatischen Aufladungen treten, wie bereits beschrieben, als ein elektrischer Störimpuls, insbesondere beim Aufsetzen der Rollen auf den Schlauch, auf. Trockene Umgebungsluft kann dieses Problem verstärken. Als unzureichende Ansätze zur Beseitigung von derartigen Aufladungen sind Antistatiksprays bekannt. Auch eignen sich aus Gründen der Biokompatibilität Schläuche aus speziellen, z.B. metallhaltigen Materialien nicht zur Reduzierung der Aufladungen.

Zwar können manche EKG-Geräte durch Einsatz von entsprechenden Filtern diese Störungen, und insbesondere die Störimpulse, herausfiltern, aber in speziellen Anwendungen, wie z.B. bei der Überprüfung von Herzschrittmachersignalen können derartige Filter nicht eingesetzt werden, da die zu messenden Herzschrittmachersignale stark den Störsignalen ähneln. Es kann, wie unter US 3,580,893, direkt an einen Schlauch ein elektrisches Erdungskabel zur Verhinderung von Aufladungen angebracht werden. Ferner sind aus WO 2004/109206 A1 Schlauchverbinder mit galvanischen Kontakten zur Erdung bekannt. Oder es können Erdungsverbinder - wie unter WO 2009/044220A1 offenbart - an den entsprechenden Schläuchen angebracht werden. Dieser Stand der Technik hat den Nachteil, dass er weniger auf die Entstehung, sondern mehr auf die Weiterleitung und schädliche Wirkung der Ladungen gerichtet ist. In der US 5,127,907 wird dabei der Ansatz gewählt, dass Teile, welche in einer Relativbewegung zueinander stehen, aus ähnlichen Materialien gefertigt werden. Nachteilig erweist sich bei den bekannten Lösungen auch der zusätzliche Aufrüstaufwand und die zusätzlichen Kosten.

Aus der US 2005/0010077 A1 ist ein Arterien-Arterien Shunt für eine pumpenassistierte Myokardrevaskularisation ohne einen kardiopulmonalen Bypass offenbart, welcher an jedem Ende einen kurzen Abschnitt eines kardiopulmonalen Bypassschlauches sicher aufnimmt, mit einem ventilierten Kanülenadapter.

Aus der US 2008/0213113 A1 ist eine Schlauchhaltevorrichtung für Rollenpumpen bekannt.

Aufgabe der vorliegenden Erfindung ist es, störende Einflüsse elektrostatischer Ladungen oder elektrischer Impulse von einer Schlauchpumpe auf elektronische Geräte zu vermeiden oder zumindest zu reduzieren.

Gelöst wird diese Aufgabe durch die Merkmale des Anspruchs 1. Bevorzugte Ausführungsformen ergeben sich aus den abhängigen Ansprüchen.

Erfindungsgemäß wird dabei eine Schlauchpumpe zur Verwendung in der Medizintechnik bereitgestellt, mit einem Stator und einem Rotor, wobei der Stator ein Rollbett aufweist, welches dem Kontaktbereich zu einem aufgenommenen Schlauch entspricht und am Rotor sind Rollelemente vorgesehen, die geeignet sind, einen zwischen dem Rollbett und den Rollelementen aufgenommenen Schlauch zu verschließen. Dabei weist das Rollbett zumindest teilweise eine elektrisch leitende Oberfläche zur Reduzierung und/oder Vermeidung von elektrostatischen Aufladungen des Schlauchs auf. Die Rollelemente können dabei Rollen oder Kugeln sein und das Rollbett kann auch mehrere getrennte Bereiche der leitenden Oberfläche aufweisen. In dem Bereich, welcher von einem Rollelement überrollt wurde, hat der Schlauch aufgrund innerer Rückstellkräfte die Tendenz sich wieder in seine ursprüngliche zylindrische Form zurückzuverformen und bei dieser Verformung heben Bereiche des Schlauchs von dem Rollbett ab und es wurde erkannt, dass aufgrund eines mangelnden Ladungsaustauschs in diesem Trennbereich elektrostatische Ladungen auf der Schlauchoberfläche entstehen können. Durch eine metallische Oberfläche des Rollbetts wird der Ladungsaustausch unterstützt und die Bildung von entsprechenden elektrostatischen Ladungen verhindert oder zumindest reduziert.

Durch diese Ausführungsform sind keine zusätzlichen für den Anwender sichtbaren Vorrichtungen an der Schlauchpumpe und/oder am Schlauchsystem notwendig, um die elektrostatische Aufladung zu verhindern. Das Bedienpersonal muss keine zusätzlichen Aufrüstschritte durchführen und hat einen unveränderten optischen Eindruck von der Schlauchpumpe und dem medizinischen Gerät, in welches die Schlauchpumpe integriert sein kann.

Weiterführend ist an der elektrisch leitenden Oberfläche ein Potentialausgleich, insbesondere in Form einer Erdung, vorgesehen. Dieser Potentialausgleich kann dadurch geschehen, dass empfindliche elektronische Geräte, welche sich in der Nähe der Schlauchpumpe befinden und gestört werden könnten, z.B. durch einen elektrischen Leiter, auf das gleiche elektrische Potential wie die Schlauchpumpe und insbesondere dessen Rollbett gebracht werden. Auch kann durch eine Erdung in das elektrische Versorgungsnetz ein Potentialausgleich geschaffen werden, welcher einen freien Ladungsausgleich erlaubt und somit eine Aufladung der Schlauchoberfläche verhindert oder zumindest effektiv reduziert. Ein vorzugsweise zwischen den Potentialausgleich und der elektrisch leitenden Oberfläche vorgesehener elektrischer Widerstand begrenzt den Ableitstrom und verhindert so eine Weiterleitung von Störsignalen mittels der Erdung an andere dort angeschlossene Geräte.

Ferner weisen vorzugsweise auch die Rollelemente eine elektrisch leitende Oberfläche auf, welche bei zylindrischen Rollen die Mantelfläche ist, und es besteht von diesen Oberflächen ein elektrisch leitender Kontakt zu der elektrisch leitenden Oberfläche des Rollbetts. Auf diese Weise liegt zu beiden Seiten des zwischen dem jeweiligen Rollelement und dem Rollbett befindlichen Schlauchs das gleiche elektrische Potential, so dass hierdurch die Bildung von elektrostatischen Ladungen auf dem Schlauch weiter reduziert wird.

In einer weiteren Ausgestaltung der Erfindung weist das komplette Rollbett eine durchgehende elektrisch leitende Oberfläche auf. Prinzipiell kann das Rollbett nur teilweise, also insbesondere abschnittsweise eine leitende Oberfläche aufweisen, aber eine entsprechende durchgehende leitende Oberfläche verbessert den Ladungsausgleich. Vorteilhaft ist insbesondere, dass dadurch bereits die Bildung von elektrostatischen Ladungen verhindert (oder zumindest reduziert) wird, was deutlich effektiver ist, als möglicherweise entstandene Ladungen abzuführen oder auszugleichen. Auch wird dadurch sichergestellt, dass keine Absätze und/oder Unebenheiten des Rollbetts entstehen, welche den Abrollvorgang der Rollelemente und so die Pumpwirkung verschlechtern und sich nachteilig auf die Lebensdauer des Schlauchs auswirken können.

Konstruktiv kann die Schlauchpumpe als Schlauchrollenpumpe ausgeführt sein, d.h. das Rollbett weist eine zylindrisch nach innen gerichtete Oberfläche auf und die Rollelemente sind dabei als Rollen ausgeführt. Schlauchrollenpumpen sind kostengünstig zu fertigen und weisen gute Eigenschaften genauer Dosierbarkeit auf. Alternativ dazu kann die Schlauchpumpe auch kugelförmige Andruck- oder Rollelemente aufweisen, wie es in einer nachfolgenden Ausführungsvariante näher beschrieben wird.

Ferner kann die zylindrische Oberfläche des Rollbetts an ein oder zwei Stellen Freimachungen aufweisen, um als Ein- oder Ausführung für den Schlauch aus der Schlauchpumpe zu dienen. So wird eine knickfreie Schlauchführung ermöglicht.

In einer weiterführenden Ausgestaltung wird der Stator als ein Spritzgussteil hergestellt und die genannte elektrisch leitende Oberfläche ist die Oberfläche eines umspritzten metallischen Einsatzteils oder einer umspritzten Metallfolie. Das Umspritzen eines Metallteils ermöglicht eine gute mechanische Verbindung von dem Metall mit dem Kunststoff. Da der Kunststoff des Spritzgussteils aus einem elektrisch nicht leitenden Kunststoff gefertigt wird, werden die Ladungen, welche an dem Rollbett auftreten können, nicht über den Kunststoff auf andere Bereiche der Pumpe und/oder ein entsprechendes medizinisches Gerät übertragen. Dabei ist das Kunststoffmaterial des Stators elektrisch nicht leitend, so dass die Ladungen sich nicht über den Stator ausbreiten können.

Auch kann weiterbildend das Einsatzteil eine zylindrische Grundform mit einem Flansch aufweisen, welcher vorzugsweise komplett umlaufend und radial nach innen ausgerichtet ist. Diese Geometrie entspricht in etwa einer Art Dose mit einem Boden, der der zylindrischen Grundform Verformungssteifigkeit verleiht. Dabei ist vorteilhafter Weise konzentrisch im Boden eine Öffnung vorgesehen, durch welche die Antriebsachse des Rotors geführt werden kann. Alternativ kann der Flansch auch nach außen gerichtet sein, d.h. wenn das Einsatzteil umspritzt wird, so zeigt dieser Flansch in Richtung des Kunststoffs und sorgt für eine gute Metall-Kunststoffverbindung.

In einer alternativen Ausführungsform kann die bezeichnete elektrisch leitende Oberfläche des Rollbetts auch durch eine leitende Beschichtung eines zuvor erstellten Spritzgussteils erreicht werden. Das Rollbett soll eine hohe Rundheit aufweisen, welche mit modernen Spritzgussmaschinen auch gut zu erzielen ist. Eine Beschichtung eines so hergestellten Spritzgussteils, wie etwa eine galvanische Beschichtung oder eine Beklebung, behält die Oberflächengenauigkeit und insbesondere die Rundheit bei. Auch kann ein elektrisch leitendes Metallteil, wie z.B. ein Edelstahlblech in eine entsprechend angespritzte Aufnahme der Schlauchpumpe eingesteckt werden, was ein einfacher und im Vergleich zu einem Umspritzen kostengünstiger Fertigungsvorgang ist und durch die Andruckkräfte der Rollelemente wird sich dieses Metallteil gut an die spritzgegossene zylindrische Oberfläche des Rollbetts anschmiegen. Ferner kann ein medizinisches Gerät eine entsprechende Schlauchpumpe umfassen, wobei das Gerät eine einem Benutzer zugewandte Bedien- und Funktionsfront aufweist, welche als ein Spritzgussteil geformt ist und dabei ist die Schlauchpumpe mit dem Rollbett einstückig in die Bedien- und Funktionsfront integriert. Hierdurch wird eine ganzheitliche Front des Geräts geschaffen und entsprechend ein ansprechendes Gesamtdesign.

Anhand der Zeichnung wird die Erfindung nachstehend eingehend erläutert. Es zeigen:
- Fig. 1: eine Prinzipskizze einer Schlauchrollenpumpe,
- Fig. 2: eine Draufsicht auf eine entsprechende Schlauchrollenpumpe,
- Fig. 3: eine Variante eines Einsatzteils und
- Fig. 4: eine weitere Variante des Einsatzteils.

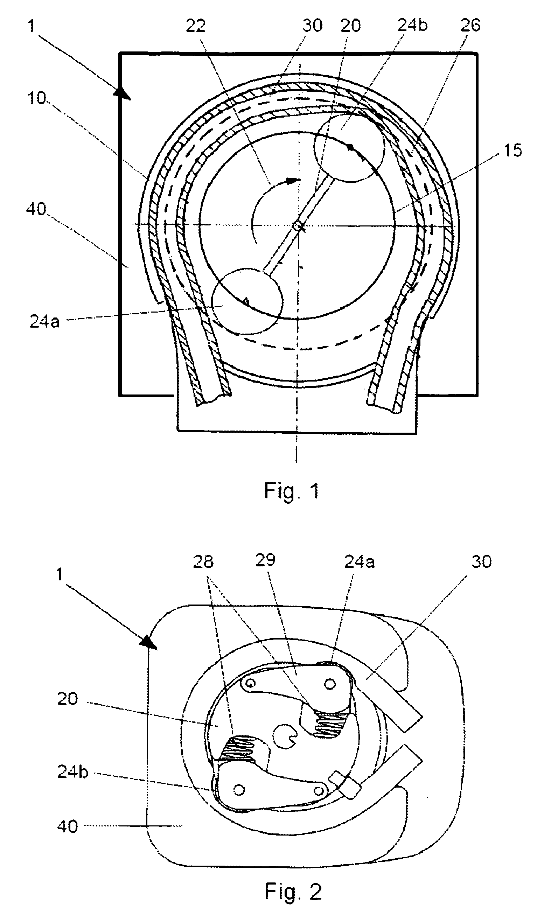

Fig. 1 zeigt das Prinzip einer Schlauchrollenpumpe. Hierbei ist ein Schlauch 30 in eine zylindrische Aufnahme eines Einsatzteils 10 eingesetzt und steht dabei umlaufend mit ungefähr 270° in Kontakt zu der Innenseite des Einsatzteils. Diese Fläche wird nachfolgend auch als Rollbett 12 bezeichnet. Koaxial zu dem Einsatzteil 10 ist ein Rotor 20 mit zwei Antriebsarmen angeordnet, an denen je ein Rollelement 24a, 24b in Form einer zylindrischen Walze drehbar gelagert ist. Der Rotor ist entsprechend der Antriebsrichtung 22 über einen Motor (nicht dargestellt) angetrieben. Dabei drückt das obere Rollelement 24b derart radial nach außen gegen den Schlauch 30, dass dieser sich lokal verschließt. Wenn nun der Antrieb den Rotor weiterbewegt, bewegt sich diese verschlossene Stelle im Uhrzeigersinn. Dabei kommt das untere Rollelement 24a in Eingriff mit dem Schlauch und verschließt diesen entsprechend und so wird das Fluidvolumen zwischen beiden verschlossenen Stellen in Längsrichtung des Schlauchs gefördert. Der Schlauch ist dabei ortsstabil zu dem Einsatzteil 10 und dessen Rollbett 12 und es findet eine Abrollbewegung zwischen den Rollelementen 24a, 24b und dem Schlauch statt. Der Außenrollradius 26 der Rollelemente ist dabei so bemessen, dass er im Wesentlichen um die doppelte Wandstärke des Schlauchs 30 kleiner als der Innenradius des Einsatzteils 10 ist, um so den Schlauch hinreichend gut zu verschließen, ohne ihn zu sehr zu quetschen.

Zur Realisierung einer gewünschten radialen Andruckkraft von den Rollelementen 24a, 24b gegen den Schlauch 30 kann, wie in Fig. 2 dargestellt, auch jeweils eine Druckfeder 28 vorgesehen sein, welche sich am Rotor 20 abstützt und über einen Rollenarm 29 die Rollen 24a, 24b entsprechend nach außen drückt. Jeder der beiden Rollenarm 29 ist am Rotor 20 schwenkbar gelagert, weist eine Andruckfläche für die Druckfeder 28 und auch eine Lagerung für eines der Rollen 24a, 24b auf. Es kann auch eine höhere Anzahl an Rollelementen verwendet werden.

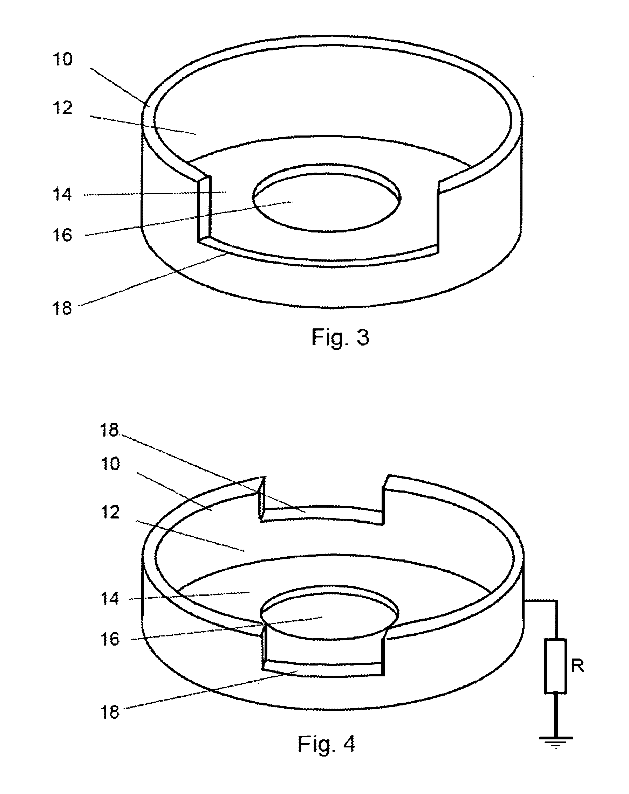

Das Einsatzteil 10 ist dabei als eine Art Dose aus einem metallischen Werkstoff, wie z.B. Edelstahlbleich, ausgeführt. Das heißt, es weist eine zylindrische Grundform auf und wird zur einen axialen Seite von einem Flansch 14 begrenzt, welcher sich radial nach innen erstreckt und eine koaxiale Öffnung 16 für die Antriebsachse des Rotors 20 frei lässt. Die zylindrische Stirnseite des Flanschs 14 ist in Fig. 1 und Fig. 3 mit dem Innendurchmesser des Flanschabschlusses 15 dargestellt. Durch eine Freimachung 18, welche an der zylindrischen Grundform des Einsatzteils 10 vom Flansch entfernt vorgesehen ist, wird gemäß Fig. 1 der Schlauch in den Innenbereich des Einsatzteils 10 geleitet. Da die Freimachung gut 90° des Umfangs aufweist, beträgt die Rolllänge des Rollbetts knapp 270°. Für die Förderfunktion der Schlauchpumpe muss stets zumindest ein Rollelement den Schlauch verschließen und so würde bei zwei Rollelementen eine Rolllänge von 180° ausreichen.

Alternativ zu der Ausführung in Fig. 3 kann der Flansch 14 auch radial nach außen zeigen oder es kann zusätzlich zu dem in Fig. 3 dargestellten Flansch 14 ein weiterer nach außen gerichteter Flansch am anderen Ende des Einsatzteils vorgesehen sein.

Einleitend wurde der technische Effekt beschrieben, dass bei herkömmlichen Schlauchpumpen bei dem Abrollen der Rollen gegen den Schlauch, dem Abheben des Schlauchs von seinem Rollbett und v.a. auch beim Aufsetzen der jeweiligen Rolle auf den Schlauch im Einführungsbereich in die Schlauchpumpe triboelektrische Aufladungen und Störimpulse erzeugt werden. Diese werden insbesondere bei dem Kontakt von Kunststoffoberflächen zueinander erzeugt.

Vorliegend ist das Einsatzteil 10 aus einem metallischen Werkstoff gefertigt und so weist das Rollbett, also die zylindrische Innenfläche des Einsatzteils, eine elektrisch leitende Oberfläche auf. Hierdurch wird der Effekt der Ladungstrennung und die entsprechende Aufladung stark reduziert oder vermieden, da durch den freien Ladungsaustausch an der leitenden Oberfläche die Bildung von einem lokalen Ladungsüberschuss oder Ladungsmangel verhindert wird. Ferner können auch die Rollelemente eine elektrisch leitende Oberfläche aufweisen und hierdurch werden auch an dem Schlauch-Rollelementkontakt entsprechende Aufladungen reduziert und/oder vermieden.

Auch kann eine elektrisch leitende Verbindung von dem Rotor 20 mit dessen Rollen 24a, 24b zu dem Rollbett vorgesehen sein, wie etwa durch einen Schleifkontakt an der Antriebsachse des Rotors 20, der elektrisch leitend mit dem Einsatzteil 10 verbunden ist (nicht dargestellt) und so wird dieser Effekt der Vermeidung der Aufladungen noch verstärkt.

In der hier beschriebenen Ausführungsform ist das gesamte Rollbett elektrisch leitend ausgeführt. Es tritt aber bereits eine deutliche Reduzierung der Störimpulse auf, wenn das Rollbett nur im Ein- und Ausführungsbereich des Schlauchs 30 mit leitenden und geerdeten Abschnitten versehen ist. Ladungen, welche sich auf dem Äußeren des Schlauchs befinden oder entsprechende Störimpulse können durch die Erdung abgeführt werden.

Das Einsatzteil 10 kann in einem Tiefziehvorgang kostengünstig mit einer genauen Rundheit und mit einer glatten Oberfläche aus einem Metallblech hergestellt werden. Dieses Einsatzteil wird in ein Spritzgusswerkzeug eingesetzt. Im Spritzgusswerkzeug wird das Einsatzteil umspritzt und bildet eine formschlüssige und feste Verbindung mit dem umspritzten Körper der Schlauchpumpe. Auch kann die Schlauchpumpe in die Frontplatte des entsprechenden medizinischen Geräts integriert sein, um für den Bediener gut erreichbar zu sein. In diesem Fall wird das Einsatzteil in der entsprechenden Frontplatte umspritzt.

Alternativ kann das Einsatzteil auch eine Metallfolie sein, welche ringförmig in das Spritzgusswerkzeug eingelegt und umspritzt werden kann.

Alternativ kann die metallische Oberftäche des Rollbetts auch nachträglich auf ein gespritztes Kunststoffformteil aufgebracht werden. Für derartige leitende Beschichtungen kann die MID-Technologie (Molded Interconnection Device) verwendet werden. Diese Technologie wird zwar primär für die Erzeugung von dreidimensionalen Leiterbahnen für elektrische Schaltkreise verwendet, aber es ist auch möglich auf diese Weise durchgehende leitende Flächen zu erzeugen. Dabei wird ein entsprechend beschichtbarer Kunststoff verwendet, welcher galvanisch mit einer entsprechenden Metallschicht versehen wird. Wenn in einem Zweikomponentenspritzgussverfahren dieser beschichtbare Kunststoff ausschließlich im Bereich des Rollbetts verwendet wird, so kann für die Front des Geräts ein anderes Material zum Einsatz kommen. Auch kann eine Edelstahlfolie oder Edelstahlblech in die zylindrische Aufnahme eingeklemmt und/oder eingeklebt werden.

Das Einsatzteil 10 (siehe Fig. 3) weist eine Freimachung 18 auf, welche gemäß Fig. 1 sowohl zur Ein- und Ausführung des Schlauches dient, so dass die Rolllänge, also die Andruckfläche des Schlauchs im Rollbett, 270° beträgt. Alternativ kann ein Einsatzteil gemäß Fig. 4 verwendet werden, welches an gegenüberliegenden Stellen zwei kleinere Freimachungen 18 aufweist. Dieses Einsatzteil kann bei einer Schlauchpumpe verwendet werden, bei der der Schlauch auf einer ersten Seite in den zylindrischen Bereich eingeleitet wird, dort über anderthalb Umläufe geführt und auf einer zweiten gegenüberliegenden Seite die Pumpe verlässt. Auch kann der Schlauch mit mehr Umläufen in der Pumpe geführt werden.

Fig. 4 zeigt schematisch einen Widerstand R, welcher elektrisch zwischen das Einsatzteil 10 und einem Potentialausgleich oder insbesondere einer Erdung geschaltet ist. Dieser Potentialausgleich kann auch mit dem Rotor 20 verbunden sein. Der Widerstand R bewirkt eine Begrenzung des Ableitstroms des Störsignals. Wenn ein empfindliches Gerät an dem Potentialausgleich oder der Erdung angeschlossen ist, so wird auf diese Weise verhindert, dass Störsignale über den Potentialausgleich an dieses Gerät weitergeleitet werden.

Bislang wurde im Ausführungsbeispiel eine Schlauchrollenpumpe beschrieben, welche Rollen als Roll- oder Andruckelemente verwendet. Die Erfindung ist entsprechend auf Schlauchpumpen mit kugelförmigen Rollelementen anwendbar. Eine derartige Schlauchpumpe ähnelt einem Radialkugellager, wobei dessen äußerer Laufring der Stator ist, welcher umlaufend eine konkave Vertiefung aufweist, in welcher der Schlauch liegt, der von vorzugsweise zwei nach außen drückenden Kugeln sukzessiv lokal verschlossen wird. Der Antrieb der Pumpe erfolgt direkt an den Kugeln, welche vergleichbar zu einem Radialkugellager in einem Käfig geführt sind.

## Patentansprüche

1. Schlauchpumpe (1) zur Verwendung in der Medizintechnik mit einem Stator (40) und einem Rotor (20), wobei der Stator (40) ein Rollbett (12) aufweist, welches dem Kontaktbereich zu einem aufgenommenen Schlauch (30) entspricht und am Rotor (20) sind Rollelemente (24) vorgesehen, die geeignet sind, einen zwischen dem Rollbett (12) und den Rollelementen (24) aufgenommenen Schlauch zu verschließen wobei das Rollbett (12) zumindest teilweise eine elektrisch leitende Oberfläche zur Reduzierung und/oder Vermeidung von elektrostatischen Aufladungen des Schlauchs (30) aufweist, **dadurch gekennzeichnet, dass**
die elektrisch leitende Oberfläche (12) einen Potentialausgleich, insbesondere in Form einer Erdung, aufweist.

2. Schlauchpumpe gemäß Anspruch 1, **dadurch gekennzeichnet, dass** zwischen dem Potentialausgleich und der elektrisch leitenden Oberfläche (12) ein elektrischer Widerstand (R) vorgesehen ist.

3. Schlauchpumpe gemäß Anspruch 1 oder 2, **dadurch gekennzeichnet, dass** die Rollelemente (24) eine elektrisch leitende Oberfläche aufweisen und dass von diesen Oberflächen ein elektrisch leitender Kontakt zu der elektrisch leitenden Oberfläche des Rollbetts (12) besteht.

4. Schlauchpumpe gemäß einem der vorangegangenen Ansprüche, **dadurch gekennzeichnet, dass** das komplette Rollbett (12) eine durchgehend elektrisch leitende Oberfläche aufweist.

5. Schlauchpumpe gemäß einem der vorangegangenen Ansprüche, **dadurch gekennzeichnet, dass** das Rollbett (12) eine zylindrisch nach innen gerichtete Oberfläche aufweist und die Rollelemente (24) als Rollen ausgeführt sind.

6. Schlauchpumpe (1) gemäß einem der vorangegangenen Ansprüche, **dadurch gekennzeichnet, dass** die Kontur des Rollbetts an ein oder zwei Stellen Freimachungen (18) aufweist, um als Ein- oder Ausführung für den Schlauch aus der Schlauchpumpe zu dienen.

7. Schlauchpumpe gemäß einem der vorangegangenen Ansprüche, **dadurch gekennzeichnet, dass** der Stator (40) aus einem elektrisch nicht leitenden Kunststoff in einem Spritzgussprozess hergestellt wurde und die elektrisch leitende Oberfläche die Oberfläche eines umspritzten metallischen Einsatzteils (10) oder einer umspritzten Metallfolie ist.

8. Schlauchpumpe gemäß Anspruch 7, **dadurch gekennzeichnet, dass** das Einsatzteil (10) eine zylindrische Grundform mit einem Flansch (14) aufweist, welcher vorzugsweise komplett umlaufend und radial nach innen ausgerichtet ist.

9. Schlauchpumpe gemäß einem der Ansprüche 1 bis 6, **dadurch gekennzeichnet, dass** die elektrisch leitende Oberfläche durch eine lokale leitende Beschichtung eines Spritzgussteils erzeugt wurde.

10. Medizinisches Gerät zur extrakorporalen Blutbehandlung, insbesondere zur Dialyse und/oder eine Infusions- oder Transfusionsvorrichtung, mit einer Schlauchpumpe (1) nach einem der vorangegangenen Ansprüche.

11. Medizinisches Gerät gemäß Anspruch 10, wobei das Gerät eine einem Benutzer zugewandte Bedien- und Funktionsfront aufweist, welche als ein Spritzgussteil geformt ist und wobei die Schlauchpumpe mit dem Rollbett einstückig in die Bedien- und Funktionsfront integriert ist.

## Claims

1. A hose pump (1) for use in medical technology, having a stator (40) and a rotor (20), wherein the stator (40) has a rolling bed (12) which corresponds to the contact area with a hose (30) that is included, and roller elements suitable for closing a hose accommodated between the rolling bed (12) and the rolling elements (24) are provided on the rotor (20), wherein the rolling bed (12) at least partially has an electrically conducting surface for reducing and/or preventing electrostatic charges in the hose (30), **characterized in that**
the electrically conducting surface (12) has potential equalization in the form of grounding in particular.

2. The hose pump according to Claim 1, **characterized in that** an electric resistor (R) is provided between the potential equalization and the electrically conducting surface (12).

3. The hose pump according to Claim 1 or 2, **characterized in that** the rolling element (24) has an electrically conducting surface and there is an electrically conducting contact from these surfaces to the electrically conducting surface of the rolling bed (12).

4. The hose pump according to any one of the preceding claims,**characterized in that** the complete rolling bed (12) has a continuous electrically conducting surface.

5. The hose pump according to any one of the preceding claims, **characterized in that** the complete rolling bed (12) has a cylindrically inwardly directed surface, and the rolling elements (24) are designed as rollers.

6. The hose pump (1) according to any one of the preceding claims, **characterized in that** the contour of the rolling bed has recesses (18) in one or two locations to serve as an inlet or outlet for the hose from the hose pump.

7. The hose pump according to any one of the preceding claims, **characterized in that** the stator (40) was manufactured from an electrically non-conducting plastic in an injection-molding process, and the electrically conducting surface is the surface of a sheathed metallic insert part (10) or a sheathed metal film.

8. The hose pump according to Claim 7, **characterized in that** the insert part (10) has a cylindrical basic shape with a flange (14) which preferably runs completely circumferentially and is directed radially inward.

9. The hose pump according to any one of Claims 1 to 6, **characterized in that** the electrically conducting surface was created by a locally conducting coating on an injection-molded part.

10. A medical instrument for extracorporeal blood treatment, in particular for dialysis and/or an infusion device or a transfusion device, having a hose pump (1) according to any one of the preceding claims.

11. The medical instrument according to Claim 10, wherein the instrument has an operating and function front facing the user, said front being shaped as an injected-molded part and the hose pump with the rolling bed being integrated in one piece into the operating and function front.

## Revendications

1. Pompe péristaltique (1) s'utilisant en technique médicale, comportant un stator (40) et un rotor (20), le stator (40) présentant un banc roulant (12), qui correspond à la zone de contact avec un tuyau reçu (30), et au rotor (20) des éléments roulants (24) sont prévus qui sont aptes à fermer un tuyau reçu entre le banc roulant (12) et les éléments roulants (24), le banc roulant (12) présentant du moins partiellement une surface conductrice électrique pour réduire et/ou éviter les charges électrostatiques du tuyau (30), **caractérisée en ce que**
la surface conductrice électrique (12) présente une liaison équipotentielle, en particulier sous la forme d'une mise à la terre.

2. Pompe péristaltique selon la revendication 1, **caractérisée en ce que**, entre la liaison équipotentielle et la surface conductrice électrique (12), une résistance électrique (R) est prévue.

3. Pompe péristaltique selon la revendication 1 ou 2, **caractérisée en ce que** les éléments roulants (24) présentent une surface conductrice électrique et qu'il y a un contact conducteur électrique de ces surfaces avec la surface conductrice électrique du banc roulant (12).

4. Pompe péristaltique selon une des revendications précédentes, **caractérisée en ce que** l'ensemble du banc roulant (12) présente une surface conductrice électrique en continu.

5. Pompe péristaltique selon une des revendications précédentes, **caractérisée en ce que** le banc roulant (12) présente une surface dirigée vers l'intérieur de forme cylindrique et que les éléments roulants (24) se présentent sous forme de rouleaux.

6. Pompe péristaltique (1) selon une des revendications précédentes, **caractérisée en ce que** le contour du banc roulant présente à un ou deux endroits des échancrures (18) pour permettre l'introduction ou la sortie du tuyau de la pompe péristaltique.

7. Pompe péristaltique selon une des revendications précédentes, **caractérisée en ce que** le stator (40) a été fabriqué dans une matière plastique non conductrice électrique par procédé de moulage par injection et que la surface conductrice électrique est la surface d'une pièce insérée métallique (10) intégrée par injection ou d'un film métallique intégré par injection.

8. Pompe péristaltique selon la revendication 7, **caractérisée en ce que** la pièce insérée (10) présente une forme de base cylindrique pourvue d'une bride (14) qui est de préférence entièrement périphérique et est dirigée radialement vers l'intérieur.

9. Pompe péristaltique selon une des revendications 1 à 6, **caractérisée en ce que** la surface conductrice électrique a été créée par un revêtement conducteur local d'une pièce moulée par injection.

10. Appareil médical pour traitement sanguin extracorporel, en particulier dialyse et/ou appareil d'infusion ou de transfusion, comportant une pompe péristaltique (1) selon une des revendications précédentes.

11. Appareil médical selon la revendication 10, cet appareil présentant un front de manoeuvre et fonctionnel tourné vers l'utilisateur et qui est conformé sous forme de pièce moulée par injection, la pompe péristaltique étant intégrée en formant bloc avec le banc roulant dans le front de manoeuvre et fonctionnel.
